# EUROPEAN PATENT SPECIFICATION

(11) **EP 2 654 854 B1**
(45) Date of publication and mention of the grant of the patent: **10.10.2018**
(21) Application number: 11810868.7
(22) Date of filing: 22.12.2011
(51) Int. Cl.: A61M 5/28, A61M 5/20

(54) **AUTOINJECTORS**
AUTOINJEKTOREN
AUTO-INJECTEURS

(30) Priority: 22.12.2010 GB 201021764; 22.12.2010 US 201061426287 P
(43) Date of publication of application: 30.10.2013
(73) Proprietor: Owen Mumford Limited, Oxford, Oxfordshire OX20 1TU (GB)
(72) Inventor: WOZENCROFT, Robert, Epsom Surrey KT19 8PQ (GB)
(74) Representative: Wynne-Jones IP Limited
(86) International application number: PCT/GB2011/052562
(87) International publication number: WO 2012/085585

(56) References cited:
- EP-A1- 2 361 648
- GB-A- 2 451 663
- GB-A- 2 451 665

## Description

This invention relates to autoinjectors.

In conventional autoinjectors loads tending to prevent or restrain movement of the syringe are transmitted to the syringe through a syringe carrier which passes these loads through the flange at the rear of the syringe. This creates a number of design issues which currently have to be allowed for in terms of build up of tolerances etc in the design of such mechanisms. We have therefore designed a mechanism in which such loads are reacted through the forward shoulder of the syringe thereby obviating at least some of the problems mentioned above. We have had to overcome various problems in order to do this, not least to ensure that the autoinjector can still be assembled by inserting the syringe forwardly into the front body assembly, with the needle shield being in a position ready to be withdrawn by removal of the front cap. A requirement to provide access to a latch or grip to grip the needle shield and a load-transmitting surface to transmit load from the forward end of the syringe to the syringe carrier has posed difficulties which have been addressed in the embodiments below.

GB2451663A discloses an injection device with a coupled cap and needle shield. GB2451665A discloses an injection device with a lock to prevent forward motion of the syringe. EP2361648A1 discloses a remover for a protective needle shield.

Accordingly, in one aspect, this invention provides an autoinjector including a body, a syringe carrier supporting a syringe or cartridge with a needle at its forward end, for movement from a rearward position to a forward position to extend a needle, the syringe or cartridge having a barrel of generally cylindrical form having at a forward region thereof a forward facing abutment surface, wherein the syringe carrier is provided with a retention surface arranged to cooperate with said forward facing abutment surface on the syringe to prevent or limit forward movement of the syringe relative to the syringe carrier, the autoinjector further including a removable needle shield releasably attached to said syringe or cartridge and shrouding the needle, a cap attached to the forward end of said body and forwardly removable, said cap having a removal surface for engaging said needle shield whereby removal of said cap removes said needle shield.

Preferably, the retention surface on said syringe carrier comprises at least two opposed lugs. Preferably, the removal surfaces on said cap comprise at least two opposed removal lugs. Preferably, the retention lugs on said syringe carrier are angularly offset with respect to the removal lugs on said cap. Conveniently, the forward facing abutment surface on said syringe or cartridge comprises a shoulder region at the forward end of said barrel. Alternatively, said forward facing abutment surface may be provided on a fixing element attached to said syringe or cartridge.

Preferably, said syringe carrier retention surface is sloping, thereby to assist passage of said needle shield during assembly. Preferably, said retention surface and removable surface are formed on circumferential or part-circumferential ribs that are adjacent or abut before removal of the cap. Preferably, said retention surface comprises spaced part-circumferential portions and said removal surface is fully circumferential.

Whilst the invention has been described above, it extends to any inventive combination or sub-combination of novel features set out above, or in the following description or claims.

The invention may be performed in various ways and an embodiment thereof, with various modifications, will now be described by way of example only, reference being made to the accompanying drawings in which:
Figure 1 is a perspective view of an autoinjector in accordance with an embodiment of this invention with the first, front cap removed prior to an injection, but before removal of the second, rear cap;
Figure 2 is a view of the autoinjector with the rear assembly and front assembly separate prior to loading of a syringe in the forward assembly and being snap-fitted together;
Figure 3 is an exploded view of the front assembly;
Figure 4 is an exploded view of the rear assembly;
Figure 5 is an enlarged view of the syringe carrier;
Figure 6 is an enlarged view of the needle shroud;
Figure 7 is an enlarged view of the front body housing;
Figure 8 is an enlarged view of the spring guide;
Figure 9 is a view showing the spring guide and syringe carrier snap-fitted together;
Figure 10 is an enlarged view of the front cap/needle shield remover;
Figure 11 is a horizontal section view taken through the cap of Figure 10 on the major axis thereof;
Figure 12 is an enlarged view of the trigger button;
Figure 13 is an enlarged view of the plunger;
Figures 14(a) and (b) are transverse section views on the major and minor planes respectively of the autoinjector when in its pre-use condition;
Figures 15(a) and (b) are transverse section views on the major and minor planes respectively of the autoinjector after use;

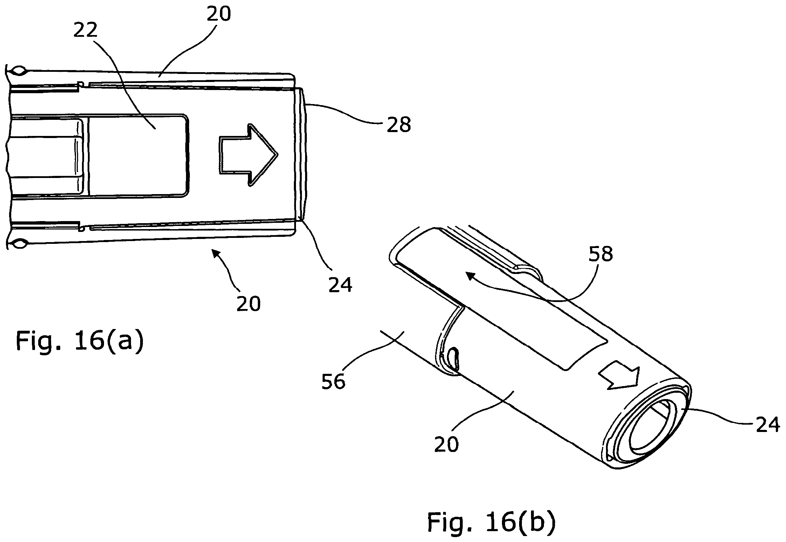

Figures 16(a) and (b) are detail views on the front end of the device showing the forwardly dished skin-contact surface;
Figure 17 is a perspective view of a first modified form of interface between the syringe, the syringe carrier, the needle shield and the front cap;
Figure 18(a) and (b) are section views through the arrangement of Figure 17 with the syringe in position;
Figure 19(a) and (b) are section views through the arrangement of Figure 17 with the syringe removed;
Figure 20 is a perspective view of a second modified form of interface between the syringe, the syringe carrier, the needle shield and the front cap;
Figures 21(a) and (b) are section views through the arrangement of Figure 20 with the syringe in position, and
Figures 22(a) and (b) are section views through the arrangement of Figure 20 with the syringe removed.

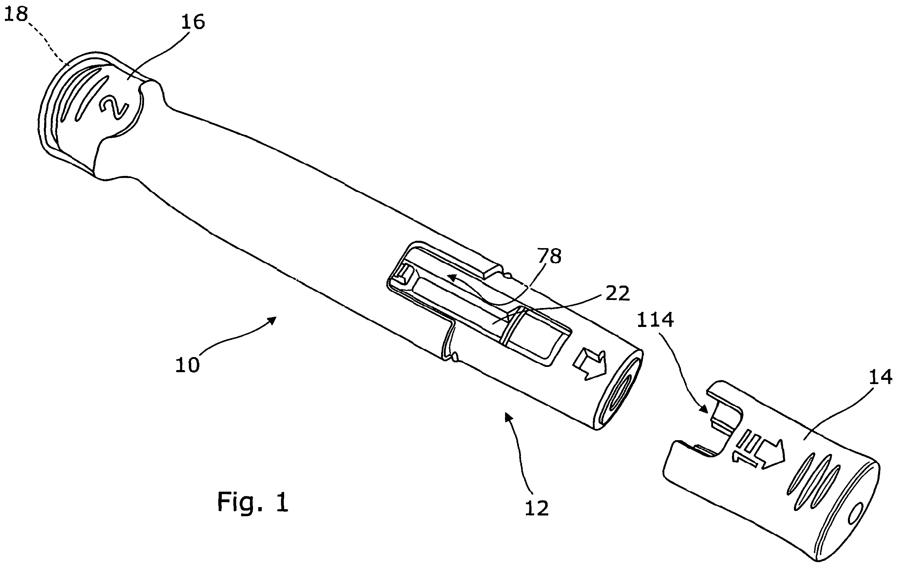

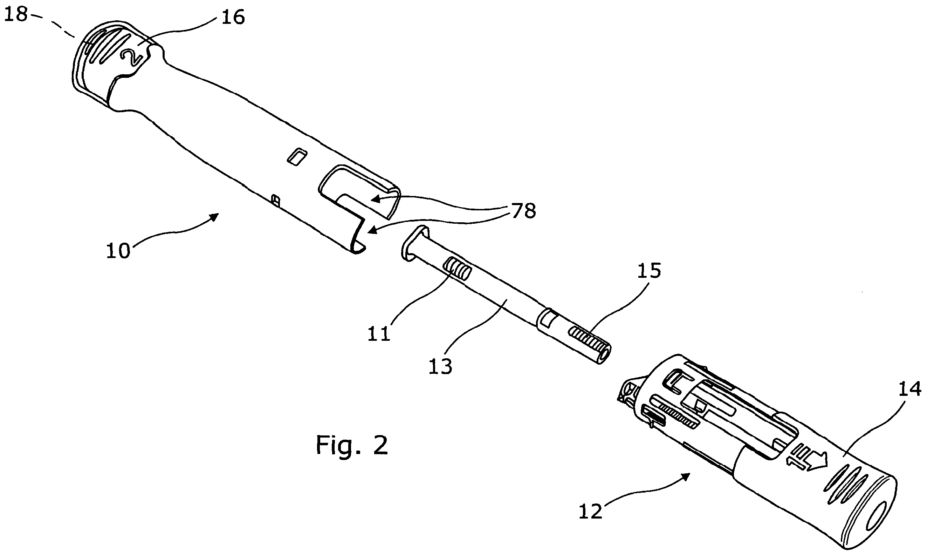

The embodiment of autoinjector illustrated in the Figures and described below is designed automatically to inject a selected dose of medicament when offered up an injection site and fired. Referring initially to Figures 1 and 2, the autoinjector comprises a rear assembly 10 containing a drive mechanism and a front assembly 12 for receiving a syringe 13 with medicament. The front and rear assemblies are snap-fitted together during manufacture. On the front end of the device is a removable cap 14 that also serves as needle shield remover as to be described below. On the rear end of the rear assembly is a rear cap 16 which includes a safety pin which prevents premature firing of the drive mechanism, the rear cap also covering the firing button 18.

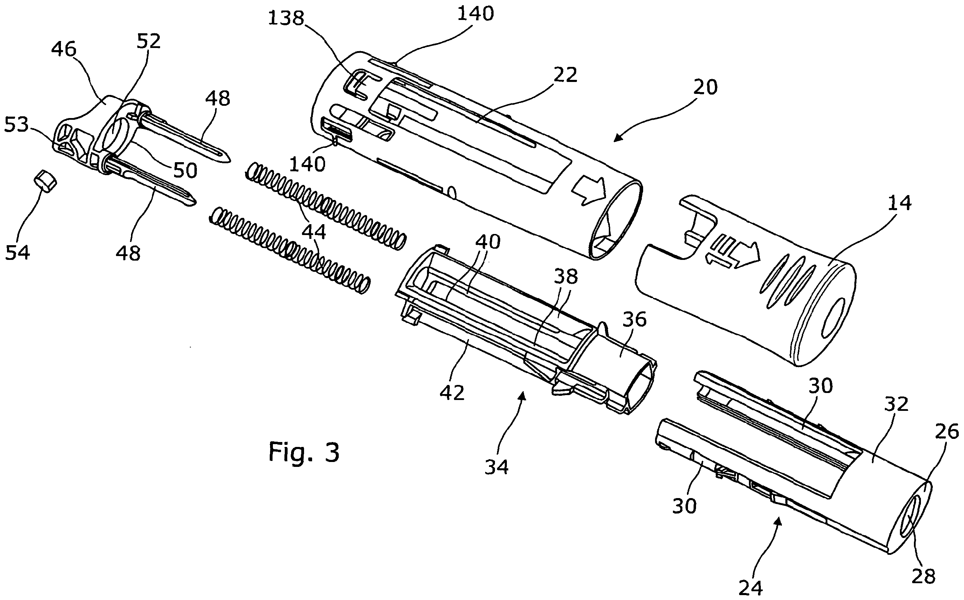

Referring now to Figure 3, the front assembly 12 comprises an outer body housing 20 of generally clear plastic material defining opposed integral viewing windows 22 through which the syringe can be viewed when the device has been assembled. The windows allow the whole of the dose volume of the syringe to be viewed. Apart from the clear plastic material of the windows 22, the body housing 20 may be opaque. Provision of a transparent window element, instead of the common arrangement of an open aperture or slot, has the advantage of preventing external access to the syringe. Also the provision of twin shroud springs spaced to either side of the longitudinal axis of the device means that the entire length of the dose volume is clearly visible without being obscured by any springs etc.

Slideably mounted within the housing 20 is a needle shroud 24 having a chamfered, conical and/or convexly curved domed front face 26 with a central aperture 28 therein to provide a forwardly dished configuration through which the needle of the syringe may project during the injection. The shroud 24 includes two rearwardly extending arms 30 of arcuate cross-section, extending back from a forward tubular section 32.

Slideably coupled to the needle shroud is a syringe carrier 34 having a forward tubular portion 36 capable of sliding telescopically inside the tubular portion 36 of the needle shroud 24. Extending rearwardly from the tubular portion 36 of the syringe carrier 34 are two arms 38 having opposed inner concave surfaces 40 for slideably receiving the barrel of a syringe and outer concave surfaces 42 for defining with convex inner arcuate surfaces on the arms 30 of the needle shroud 24, cylindrical containment spaces for a pair of shroud springs 44.

A spring guide 46 has two forwardly extending fingers 48 that pass down the centre of a respective spring 44. The spring guide 46 has an over-moulded liner 50 surrounding a circular aperture 52 through which a syringe is passed. The liner serves as a shock absorber for the syringe. The spring guide 46 is a snap fit with the rear end of the syringe carrier 34 as to be described below. The spring guide 46 has a rearwardly extending tubular portion in one side wall of which is a recess 53 for captively receiving a disc magnet 54.

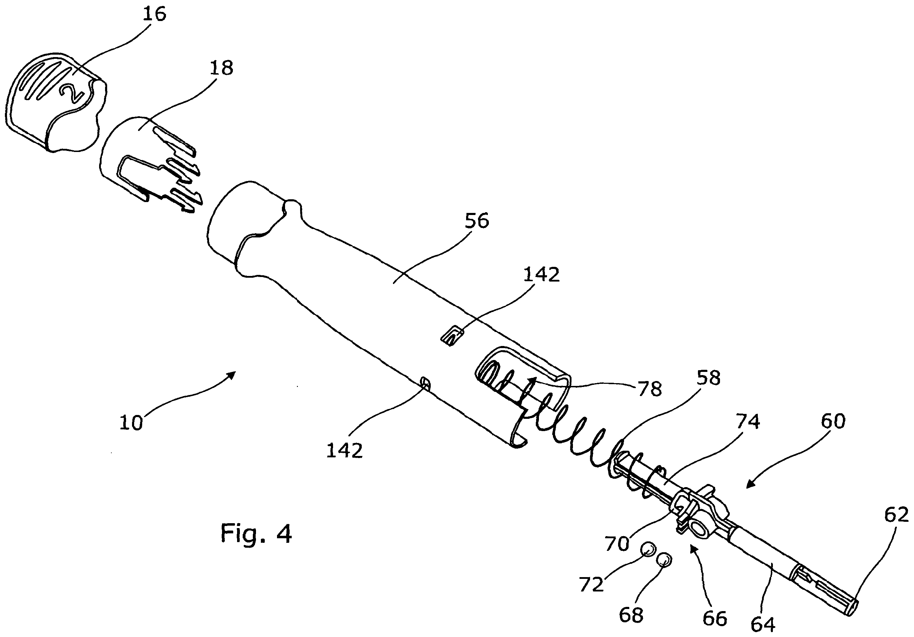

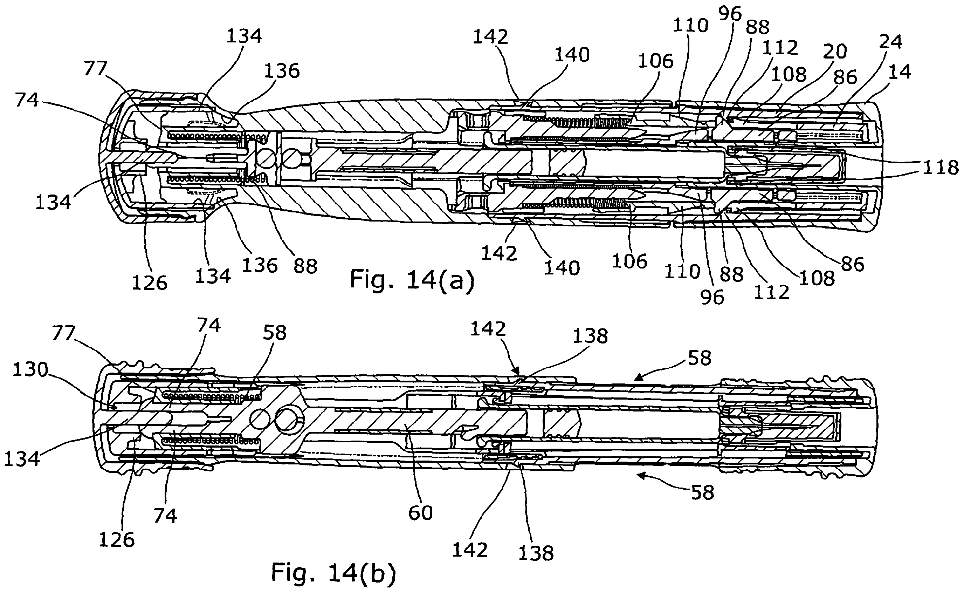

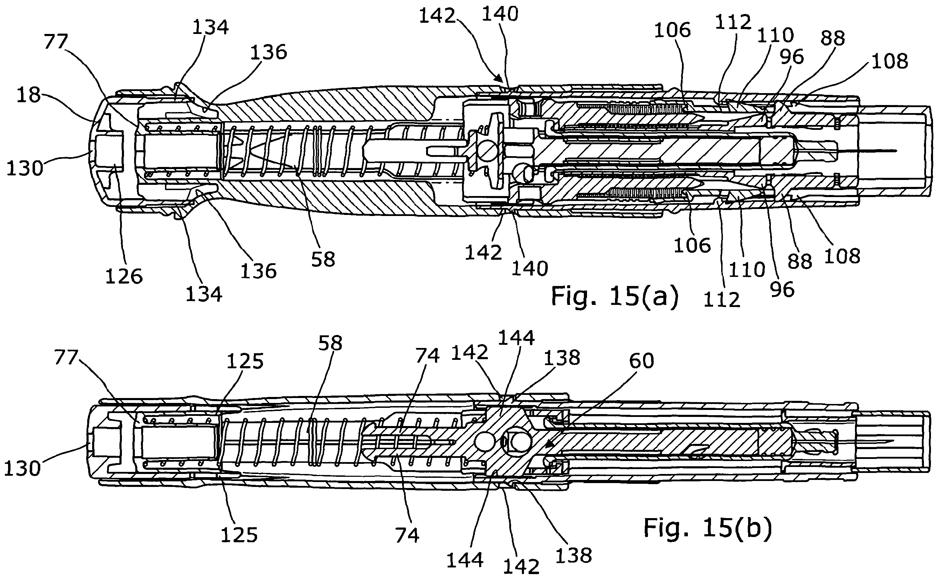

Referring now to Figure 4, the rear assembly comprises a rear body housing 56 in which is received the main drive spring 58 which acts on the rear end of a plunger 60. The plunger has a forward end 62 for engaging the piston 11 within a syringe and an over-moulded coloured indicator strip 64. To the rear of the indicator strip 64 is a transverse passage 66 in which is mounted for transverse movement a ball magnet 68. To the rear of the passage 66 is a provided a recess 70 which receives a ferro-magnetic keeper ball 72 which is fixedly disposed on the longitudinal axis of the plunger 60. The plunger 60 has two rearwardly extending split arrowhead limbs 74 with barbs 76 on the rear ends which seat around the edge of an annular catchment surface 77 in the inside of the rear body housing 56 (see Figures 14 and 15) to latch the plunger in a cocked position, with the main spring 58 compressed.

The autoinjector is of modular construction designed to allow all except two components to be the same for autoinjectors with syringes of three different fill volumes. The shape and the size of the syringe itself is standard; only the fill volume is different. The two components that vary are the rear body housing 10 and the plunger 60. The forward end of the rear body housing 52 contains opposed cut outs or slots 78 which are of variable length according to the fill volume contained in the syringe. The axial length of the slots 78 in the rear body housing 56 is proportional to the fill volume. Also the indicator position moves by the same amount so that it arrives at the same place relative to the body at the end of the plunger stroke. The plunger is also modified according to the fill volume of the syringe to locate the magnet-containing passage 66 so that, at the end of its forward stroke, it reaches the same axial position with respect to the rear body housing 56 for each fill volume. In other words, the plunger 60 and the axial length of the slots 78 are designed so that, for each of the plurality of fill volumes, the user will see prior to use in the viewing window 22 just that length of the syringe containing the dose, with the window being framed at the rear end by the slots 78. After the dose has been delivered, the indicator will be at the same forward position for each fill volume.

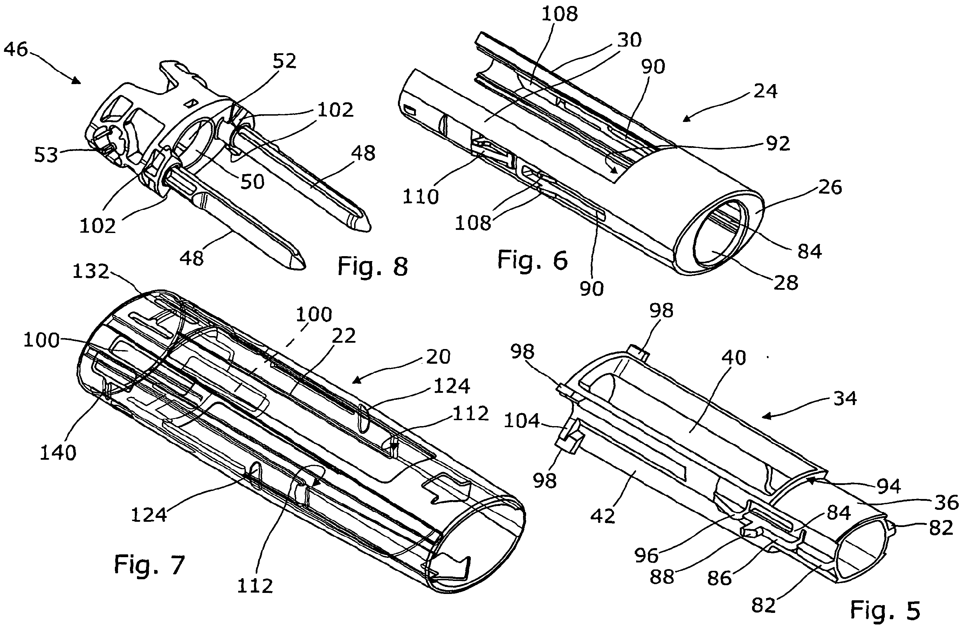

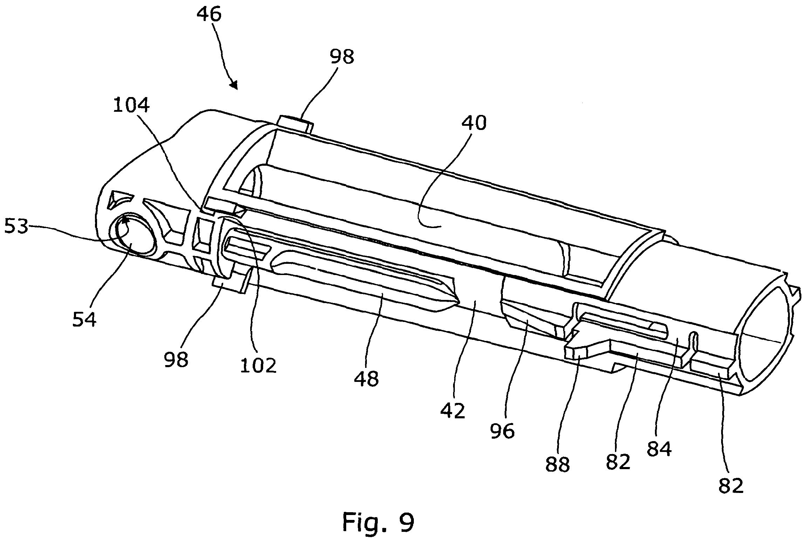

Referring now to Figures 5 to 9, the assembly of the principal components of the front assembly will be described in more detail. The syringe carrier 34 has twin linear ribs 82 provided to either side of the forward tubular portion 36. The ribs 82 run in respective channels 84 on the inside of the tubular portion 32 of the needle shroud. Immediately behind each rib 82 is a live hinge 85 from which extends back a spring finger 86 with a barb 88 with a rearwardly inclined forward surface. When the syringe carrier is assembled telescopically into the needle shroud 24, the barbs 88 project through slots 90 in the shroud 24 (see Figure 6) to limit forward movement of the shroud 24 relative to the syringe carrier 34 when the rear ends of the slots 90 contact the barbs 88. Rearward movement of the shroud 24 relative to the syringe cap is limited by a rearward shoulder 92 of the needle shroud tubular portion abutting a forward facing shoulder 94 upstanding from the rear of the tubular portion 36 of the syringe carrier 34. Rearwardly of the barbs 88 on the syringe carrier are two rearwardly facing ramp surfaces 96.

At its rear end, the syringe carrier has four lugs 98 that, when the device is assembled, run in respective slots 100 in the front body portion 20 to limit linear movement of the syringe carrier relative to the front body portion 20. Snap fitted onto the rear of the syringe carrier is the spring guide 46 as shown in Figure 8. This has snap fit tabs 102 that snap fit around walls 104 on the rear end of the syringe carrier. The tabs also form a platen surface for the shroud springs 44, with the spring guide fingers 48 passing down the centre thereof. The forward ends of the shroud springs are seated on projecting fingers 106 towards the rear of the arms 30 of the needle shroud 24. About two-thirds of the way back from the front of each slot 90 are two barbs 108 with inclined forward surfaces. Behind each slot 90, on a live hinge is a rearward barb 110, again with an inclined forward surface. The barbs 108 and 110 cooperate with respective opposed barbs 112 about a third of the way down the length of the front body housing 20 on the inner walls thereof.

The arrangement of the barbs in the pre-use position can be clearly seen in Figures 14 and 15. In the pre-use position, the barbs 108 on the needle shroud cooperate with the barbs 112 on the front body housing to prevent rearward movement of the needle shroud 24. The forward faces of the barbs 88 on the syringe carrier also cooperate with the barbs 112 on the front body housing on the forward housing to prevent forward movement of the syringe carrier 34 prior to and during removal of the front cap 14. Removing the cap removes a bracing on the barbs 88 which initially prevents inward movement of the barbs so that, when fired, the force of the drive spring causes the barbs 88 to cam past the barbs 112 on the front body housing. During operation of the device, when fired, with the needle shroud 24 held against forward movement by its contact with the skin around the injection site, the sub-assembly of the syringe 13 and the syringe carrier 34 is shifted forwardly, relative to the forward housing to a limit position defined by the lugs 98 reaching the forward ends of the slots 100. After the injection is complete, the needle shroud 24 moves forward as the skin contact pressure is removed from the surface 28 as the device is lifted clear of the skin. This allows the needle shroud to move forwardly under the influence of the shroud springs 46 so that the rear barbs 110 move forwardly and snap past the barbs 112 on the front housing 20 to prevent retraction once the needle shroud has extended. The barbs 110 are braced in this position by the underlying ramp surfaces 96 on the syringe carrier 34.

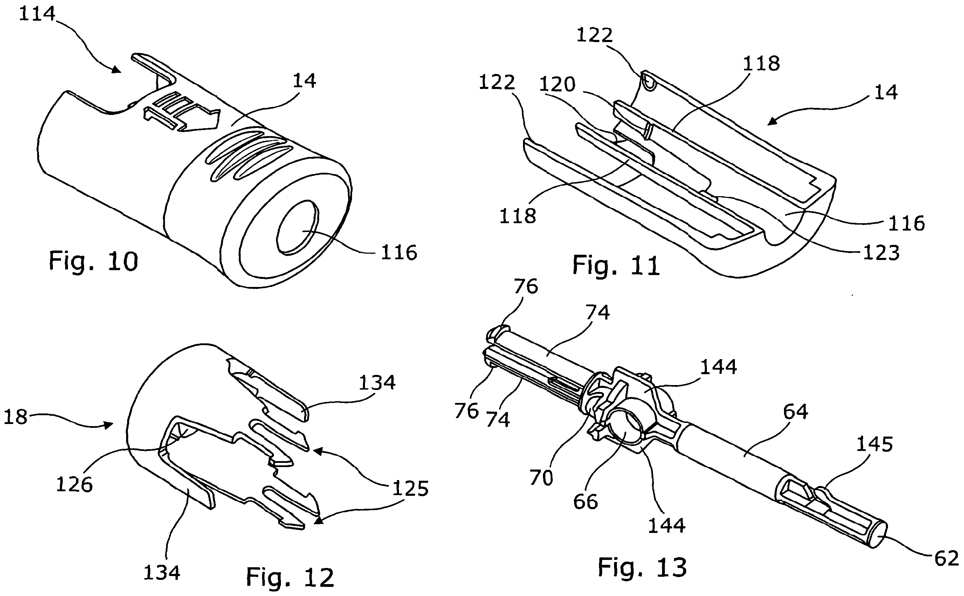

Referring now to Figures 10 and 11, the removable front cap 14 has opposed slots 114 which align with the slots 78 on the rear body housing 56, to frame the window 22 in the front body housing 20 to allow viewing of the dose volume as described above. Referring more particularly to Figure 11, the cap is elliptical in outer section and has an inner central cylindrical portion 116 extending rearwardly from which extend further two fingers 118 of arcuate cross-section disposed on the major axis of the ellipse. On the inner surface of the fingers, towards the rear ends, are respective inwardly directed barbed ribs 120 with inclined rear surfaces. As seen in Figures 14 and 15, the ribs 120 are designed to snap into a gap formed between the forward shoulder on the barrel of the syringe 13 and the rear surface of the rigid needle shield 15 or an aperture therein. When the syringe 13 is loaded into the front assembly 12 (with the cap 14 attached) during manufacture, the rigid needle shroud 15 snaps past the ribs 120 so that they lodge behind the rear edge of the needle shield 15 (or a rear edge of an aperture in the needle shield) as shown. The front cap 14 also has twin shallow scallops 122 which releasably engage pips 124 on the outer surface of the front body housing when the cap is fitted (see Figures 14 and 15).

When in the condition as supplied (Figure 14) the fingers 118 of the cap underlie the spring fingers 86 on the syringe carrier 34 and prevent these from flexing inwardly. In this condition, the fingers 118 thus brace the spring fingers 86 against inward unlatching motion. The forward end of the cylindrical portion 116 of the cap 14 is also provided with inward projections 123 aligned with the minor axis of the ellipse and which prevent forward movement of the rigid needle shield relative to the front cap 14. In this way, when the front cap 14 is withdrawn from the position shown in Figure 15, the ribs 120 pull the rigid needle shield 15 to ease it off the forward end of the syringe 13. At the same time the presence of the fingers 118 also temporarily locks the syringe carrier 34 (and thus the syringe 13) against forward movement by blocking the fingers 86 against inward movement until the needle shield is off the syringe to prevent the syringe from being pulled forwardly if there is a tight fit between the syringe and the needle shield. When the front cap is free of the device the needle shield 15 is captive in the cap 14, trapped by the ribs 120 and the inward projections 123. Orienting the ribs 120 and the inward projections 123 at 90° means that the open ended cap may be injection-moulded in a simple injection mould with a slide rather than requiring a more complex mould design.

Referring to Figures 4, 12 and 15, the firing button 18 is of elliptical form with two split arrowhead tabs 125 aligned with the minor axis, which seat behind respective ribs on the inner rear surface of the rear housing portion 56 to retain the firing button 18 on the rear of the housing and to limit rearward movement thereof. The inner rear surface of the trigger has a firing boss 126 which is of slightly smaller diameter than the outer diameter of the split arrowheads 74 on the rear of the plunger 60 so that, when the firing button 18 is pressed forwardly from the position shown, the boss squeezes the twin arrowheads 74 together to release the barbs 76 from the catchment surface 77 to free the plunger for forward movement. The firing button 18 has an aperture 130 concentric with the boss 126 through which a safety pin 134 on the rear cap 16 passes to hold the split arrowheads apart. Aligned with the major axis of the ellipse are two forwardly extending flexible biasing strips 134 which cooperate with respective bias camming surfaces 136 in the rear end of the rear housing 56, as shown in Figures 14(a) and 15(a) to provide a low friction gliding plastic-to-plastic surface contact . The camming surfaces 136 are shaped to provide a predetermined variation of resistance force with distance. The biasing strips cooperate with the curved rear portion of the camming surfaces to provide a bias force tending to restore the button to its rearmost position as defined by the split arrowhead tabs. It is desirable to provide a tactile resistance to movement and to require a few millimetres of movement before the firing boss 126 releases the plunger, to avoid premature firing. A forward portion of the camming surfaces is of shallower inclination and designed to provide a non-reversible resistance to movement after the device has been fired, thereby to trap or wedge the firing button in its forwardmost position. This gives a further useful visual cue to a user as to whether the device has been fired or not. Of course, if required the camming surface may instead be designed to return the button to its original position after firing.

The autoinjector as illustrated includes several safety features to prevent inadvertent firing and to render the device safe after use. It is also highly desirable to resist or prevent disassembly of the device after use. It will be noted from the description and Figure 2 above that the device is assembled by inserting a syringe into the syringe carrier in the front assembly and then snap-fitting the front and rear assemblies together. The snap fitting is done by means of outwardly facing sprung tabs 138, 140 on the rear of the front body housing 20 which seat simultaneously in respective apertures 142 in the rear body housing 56. One pair of tabs 138 is aligned with the minor axis and one pair 140 with the major axis of the device. It will be appreciated that, given appropriate dexterity and strength, it would be possible to press in all four of the tabs 138, 140 by poking an implement through the recesses 142 from outside and thereby disassemble the device. However, this is prevented in this embodiment by means of two fin formations 144 provided on the plunger 60 as seen in Figures 13 and 15(b). The plunger is designed so that, once the device is fired and the plunger is at its post-firing position, the fin formations 144 underlie the tabs 138 on the minor axis of the ellipse, as shown in Figure 15(b), thereby bracing them against inward deflection and preventing disassembly.

For operation, the user removes the front cap 14 and rear cap 16, thereby arming the device. The device is then offered up to the injection site to press the conical or curved front face of the needle shroud 26 against their skin. When ready, the firing button 18 is pressed, which releases the plunger 60 for forward movement under the action of the main drive spring 58. Initially, due to a sprung engagement finger 145 on the plunger, the plunger and syringe move as one forwardly to extend the needle to penetrate the flesh, with this movement continuing until the lugs 98 on the syringe carrier reach the forward end of the slots 100 on the front body housing, thereby inserting the syringe needle to the required depth. Upon arresting movement of the syringe, the sprung engagement finger 145 flexes inwardly into the bore of the syringe and the plunger continues to move, driving the piston 11 down the syringe body to expel a dose. Alternatively, in other designs of the device, the spring engagement finger may yield so that the plunger starts to move into the syringe before forward movement of the latter is arrested. In either design, when the plunger reaches its forwardmost position, the ball magnet 68 which up till now has been held in the passage 66 on the centre line of the plunger by magnetic attraction to the keeper ball 72 is attracted by the greater force provided by the disc magnet 54 held in the recess of the spring guide, accelerating towards it and impacting the magnet and/or spring guide to produce a loud audible click to indicate to the user that the injection is complete.

The user then removes the device from their skin and the release of pressure on the front end of the needle shroud 24 means that it can now extend forwardly under the influence of the twin shroud springs 44 to move forwardly to shield the needle. As it nears its forwardmost position, the barbs 110 snap past the barbs 112 on the inside of the front housing 20 thereby to prevent retraction of the needle shroud.

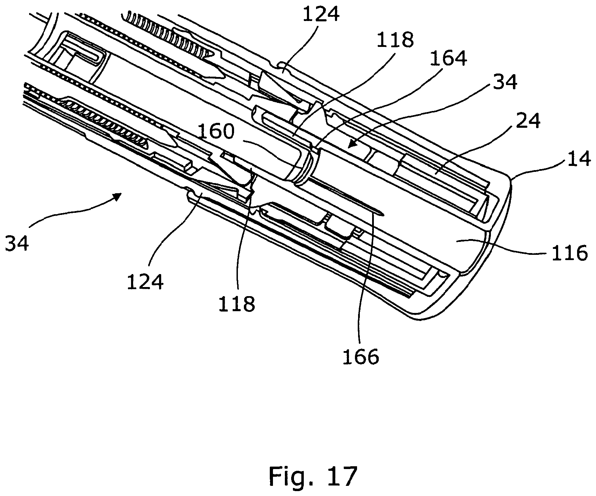

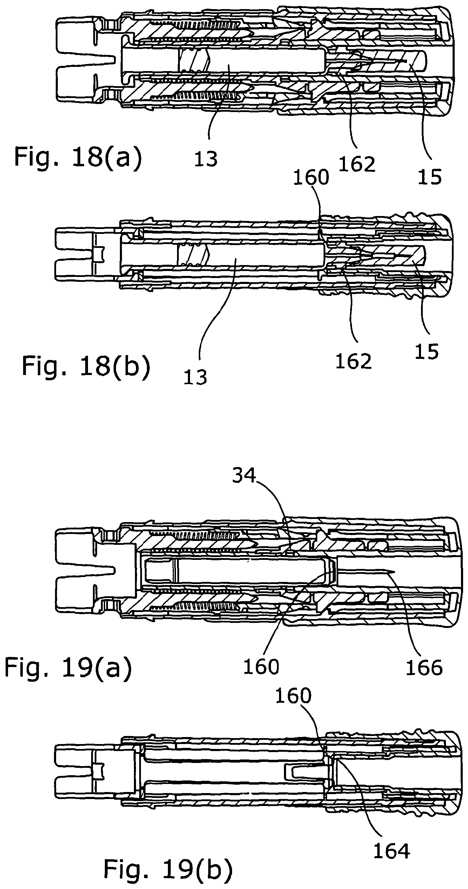

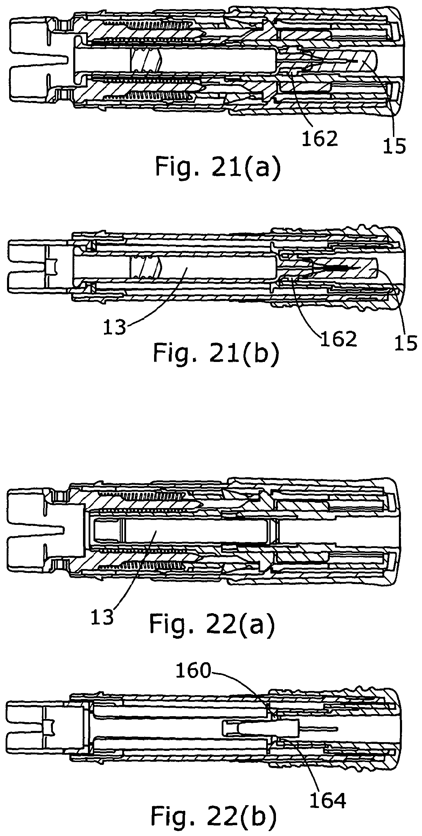

Referring now to Figures 17 to 22, these show different arrangements of interface between the forward shoulder of the syringe, the syringe carrier, the needle shield and the front cap. One of the difficulties encountered with automatic assembly of autoinjectors is that the outer diameter of the needle shield is similar to that of the syringe. Accepted assembly processes require that, once the syringe has been filled with medicament and the needle shield applied, the needle shield must not be removed during assembly. However, a common way of assembling autoinjectors is to assemble the front assembly of a device first and then to slide in the syringe forwardly, as discussed above. In the past, these two factors combined have meant that any restraining load transmitted by the syringe carrier to the syringe has been reacted to the syringe through the enlarged flange at the back end of the syringe body, not least because the front end of the syringe carrier needs to be of sufficiently wide diameter to allow the needle shield to be passed through it during assembly. Our studies have indicated that it would be beneficial instead to react the restraining loads on the syringe through the forward shoulder of the syringe, and this has meant a radical redesign of the front end of the syringe carrier and the associated region of the front cap.

The embodiments illustrated below are designed so that the syringe carrier has a load transmission surface that engages the front shoulder of the syringe but is designed to allow the needle shield to pass through, possibly with some resilient distortion. In addition to the significant constraints referred to above, another design constraint is that the front cap must be able to latch behind a surface on the needle shield so that the needle shield can be pulled off the needle to prepare the autoinjector for use by removing the cap forwardly. We have therefore designed appropriate needle shield contacting surfaces on the front cap which sit close to the reaction surface on the syringe carrier and past which the needle shield can be resiliently slipped.

Referring more particularly to Figures 17 to 19, the syringe carrier 34 is provided with two part arcuate oppositely facing supporting cups 160 which have a forward facing wall in the plane perpendicular to the axis of the device and a rearward wall which is chamfered. The radius and chamfer of the arcuate portion 160 are carefully selected such that, a syringe 13 with a needle shield 15 of soft rubber-like form (e.g. silicone rubber) can be pushed forwardly so that an annular rib 162 can squeeze past the surface 160 (assisted by the chamfer) but that the forward facing shoulder on the front end of the barrel of the syringe 13 is retained thereby. In this embodiment, the cylindrical portion of the forward cap is provided with two rearwardly extending fingers 118 as previously for the purposes described above. The cylindrical portion extends back a little further than in the arrangement of Figure 11 and a shield removal rib 164 buts up against the arcuate surface 160. Their inner radial diameters are the same and so the surface 160 also helps to cam or guide the annular rib 162 past the constriction so that it can re-expand when fully inserted, ready to be picked up and removed off the syringe when the cap is withdrawn. Two spaced ventral ribs 166 are provided to grip the needle shield 15 so that it does not fall out of the cap when the cap is removed.

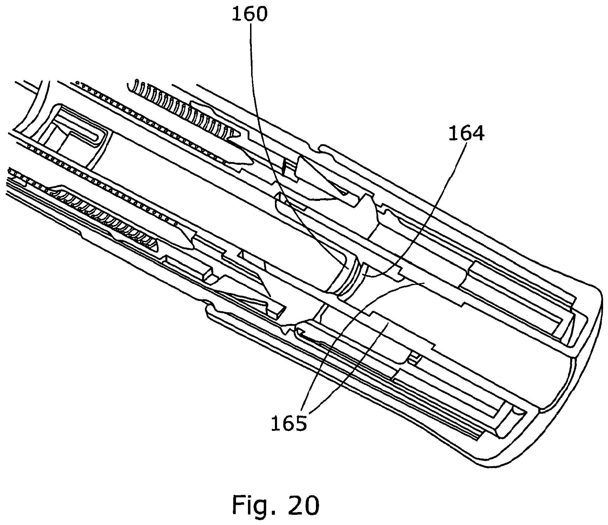

Referring to Figure 20, in this slightly modified option, instead of having a needle shield removal rib 164 that extends around 360°, the rib 164 is instead interrupted circumferentially to provide two arcuate portions which are essentially in alignment with the portions 160. In this way, there is an expansion region provided by the gaps left between the portions 164 to allow for expansion in the orthogonal direction as the needle shield is squeezed by surfaces 160 during loading of the syringe into the front assembly. Also, in this modified arrangement, the needle shield is retained in the cap by means of two opposite lugs 168 on the interior of the cylindrical portion 116 which cooperate with the annular rib 162.

## Claims

1. An autoinjector including a body (10, 12), a syringe carrier (34) supporting a syringe (13) or cartridge with a needle at its forward end, for movement from a rearward position to a forward position to extend a needle, the syringe or cartridge having a barrel of generally cylindrical form having at a forward region thereof a forward facing abutment surface, wherein the syringe carrier is provided with a retention surface (160) arranged to cooperate with said forward facing abutment surface on the syringe to prevent or limit forward movement of the syringe relative to the syringe carrier, the autoinjector further including a removable needle shield (15) releasably attached to said syringe or cartridge and shrouding the needle, a cap (14) attached to the forward end of said body and forwardly removable, said cap having a removal surface (120) for engaging said needle shield whereby removal of said cap removes said needle shield;
**characterised in that**:
the retention surface on said syringe carrier comprises at least two opposed lugs;
the removal surface on said cap comprises at least two opposed removal lugs; and
the retention lugs on said syringe carrier are angularly offset with respect to the removal lugs on said cap.

2. An autoinjector according to Claim 1, wherein the forward facing abutment surface on said syringe or cartridge comprises a shoulder region at the forward end of said barrel.

3. An autoinjector according to Claim 1, wherein said forward facing abutment surface is provided on a fixing element attached to said syringe or cartridge.

4. An autoinjector according to Claim 1, wherein said syringe carrier retention surface is sloping, thereby to assist passage of said needle shield during assembly.

5. An autoinjector according to Claim 1, wherein said retention surface and removable surface are formed on circumferential or part-circumferential ribs that are adjacent or abut before removal of the cap.

6. An autoinjector according to Claim 1, wherein said retention surface comprises spaced part-circumferential portions and said removal surface is fully circumferential.

## Patentansprüche

1. Autoinjektor mit einem Körper (10, 12), einem Spritzenträger (34) mit einer Spritze (13) oder Kartusche mit einer Nadel an ihrem vorderen Ende zur Bewegung aus einer hinteren Position in eine vordere Position zur Verlängerung der Nadel, wobei die Spritze oder Kartusche eine Trommel allgemein zylindrischer Form aufweist, die in ihrem vorderen Bereich eine nach vorne weisende Anschlagfläche besitzt, wobei der Spritzenträger des weiteren mit einer Rückhalte-Oberfläche (160) versehen ist, die so angeordnet ist, daß sie mit der nach vorne weisenden Anschlag-Oberfläche auf der Spritze zusammenwirkt, um eine Vorwärtsbewegung der Spritze in Bezug auf den Spritzenträger zu verhindern oder zu begrenzen, wobei des weiteren der Autoinjektor mit einem entfernbaren Nadelschutz (15) versehen ist, der an der Spritze oder Kartusche lösbar angebracht ist und die Nadel umhüllt, wobei ferner eine Kappe (14) an dem vorderen Ende des Körpers angebracht und nach vorne entfernbar ist, sowie eine Entfernungs-Oberfläche (120) aufweist, die mit dem Nadelschutz in Eingriff steht, wodurch das Entfernen der Kappe den Nadelschutz entfernt, **dadurch gekennzeichnet, daß** die Rückhalte-Oberfläche auf dem Spritzenträger wenigstens zwei gegenüberliegende Ansätze aufweist, und daß die Entfernungs-Oberfläche auf der Kappe wenigstens zwei gegenüberliegende Entfernungsansätze aufweist, so daß die Rückhalteansätze auf dem Spritzenträger in Bezug auf die Entfernungsansätze auf der Kappe winkelversetzt sind.

2. Autoinjektor nach Anspruch 1, **dadurch gekennzeichnet, daß** die nach vorne weisende Anschlag-Oberfläche auf der Spritze oder Kartusche an dem vorderen Ende der Trommel einen Schulterbereich bildet.

3. Autoinjektor nach Anspruch 1, **dadurch gekennzeichnet, daß** die nach vorne weisende Anschlag-Oberfläche auf einem Befestigungselement vorgesehen ist, das an der Spritze oder Kartusche angebracht ist.

4. Autoinjektor nach Anspruch 1, **dadurch gekennzeichnet, daß** die Rückhalte-Oberfläche des Spritzenträgers geneigt ist, um dadurch den Durchgang des Nadelschutzes während des Zusammenbaus zu unterstützen.

5. Autoinjektor nach Anspruch 1, **dadurch gekennzeichnet, daß** die Rückhalte-Oberfläche und die entfernbare Oberfläche auf Umfangs- oder Teilumfangsrippen ausgebildet sind, die bevor die Kappe entfernt wird, benachbart sind oder anstoßen.

6. Autoinjektor nach Anspruch 1, **dadurch gekennzeichnet, daß** die Rückhalte-Oberfläche mit Abstand getrennte Teilumfangsabschnitte aufweist, und daß die Entfernungs-Oberfläche sich vollständig um den Umfang erstreckt.

## Revendications

1. Auto-injecteur comprenant un corps (10, 12), un support de seringue (34) supportant une seringue (13) ou une cartouche avec une aiguille à son extrémité avant, pour un mouvement d'une position arrière à une position avant afin d'étendre une aiguille, la seringue ou la cartouche ayant un tube de forme généralement cylindrique ayant, à une région avant de celui-ci, une surface de butée dirigée vers l'avant, le support de seringue étant muni d'une surface de retenue (160) agencée pour coopérer avec ladite surface de butée dirigée vers l'avant sur la seringue afin d'empêcher ou limiter un mouvement de la seringue vers l'avant par rapport au support de seringue, l'auto-injecteur comprenant en outre une protection d'aiguille amovible (15) fixée de manière libérable à ladite seringue ou ladite cartouche et enveloppant l'aiguille, un capuchon (14) fixé à l'extrémité avant dudit corps et amovible vers l'avant, ledit capuchon ayant une surface de retrait (120) pour engager ladite protection d'aiguille, ce par quoi le retrait dudit capuchon retire ladite protection d'aiguille ;
**caractérisé par le fait que** :
la surface de retenue sur ledit support de seringue comprend au moins deux tenons opposés ;
la surface de retrait sur ledit capuchon comprend au moins deux tenons de retrait opposés ; et
les tenons de retenue sur ledit support de seringue sont décalés angulairement par rapport aux tenons de retrait sur ledit capuchon.

2. Auto-injecteur selon la revendication 1, dans lequel la surface de butée dirigée vers l'avant sur ladite seringue ou ladite cartouche comprend une région d'épaulement à l'extrémité avant dudit tube.

3. Auto-injecteur selon la revendication 1, dans lequel ladite surface de butée dirigée vers l'avant est prévue sur un élément de fixation fixé à ladite seringue ou ladite cartouche.

4. Auto-injecteur selon la revendication 1, dans lequel ladite surface de retenue de support de seringue est inclinée, de façon à faciliter le passage de ladite protection d'aiguille pendant l'assemblage.

5. Auto-injecteur selon la revendication 1, dans lequel ladite surface de retenue et ladite surface de retrait sont formées sur des nervures circonférentielles ou partiellement circonférentielles qui sont adjacentes ou en butée avant le retrait du capuchon.

6. Auto-injecteur selon la revendication 1, dans lequel ladite surface de retenue comprend des parties partiellement circonférentielles espacées et ladite surface de retrait est entièrement circonférentielle.
